# EUROPEAN PATENT APPLICATION

(11) **EP 2 186 467 A1**
(43) Date of publication of application: **19.05.2010**
(21) Application number: 08169332.7
(22) Date of filing: 18.11.2008
(51) Int. Cl.: A61B 5/00, A61M 5/42

(54) **Apparatus and method for determining the position of a vein or artery.**

(71) Applicant: UMC Utrecht Holding B.V., 3584 CM Utrecht (NL)
(72) Inventor: The designation of the inventor has not yet been filed
(74) Representative: van Westenbrugge, Andries

(57) **Abstract**

Apparatus and method for determining the position of a vein or artery in a member of a living being. With an IR light source (20), more particular an IR led light source, the related member such as an extremity and more particular a hand, is trans-illuminated. The IR light emerging from the member is scanned with a camera (14) and the image is produced on a display (13) adjacent to the camera. Camera (14) and display (13) are preferably mounted on a manoeuvrable arm (5,35) which can be placed in any desired position relative to the member and observer. Displacement of the camera/display unit can be effected through the presence of a grip to be operated by the hand of the observer. If the quite position is found relative to the member the hand of the observer can be used to execute the related treatment to the vein or artery.

## Description

The present invention relates to an apparatus for determining the position of a vein or artery in a member of a living being, comprising an IR source adapted to be placed at one side of a member of a living being and transferring light through such a member, a camera adapted to be placed at another side of a member of a living being, said other side being opposite to one side of a member of a living being and sensitive for radiation from said IR source, observation means for observing the image of said camera. Such an apparatus is known from WO 96/36273.

Vessel puncture is a common procedure in daily hospital/health care practice for e.g. blood withdrawal, catheter placement or to administer medicine and fluids. Normally, superficial veins and arteries are visible and easy to access. However, in a significant percentage of patients (up to 20%) the vessels are not visible and the technician or nurse has to rely on indirect signs like tactile feedback, pressure, pulsation or experience. This results in a relative high failure rate to access the vessel at the first and even secondary puncture attempts. This is a painful experience for the patient and inefficient for the procedure. If not successful after several attempts, more experienced professionals like anaesthesiologists need to be called upon, to perform the puncture which is time consuming and uncomfortable for the patient. E.g. various patient groups are notorious for being difficult to puncture: young children with baby fat (age ∼0,5 - 3 years), dark skinned patients and patients with a high body fat index and relatively tiny and deep veins. Since arteries are deeper embedded under the skin, arterial puncture is more difficult in general and needs to be performed with greater care due to the larger arterial pressure leading to complications.

The problem described is well recognized by the professionals performing the punctures and is extensively describe in literature.

Transferring light through a member of a living being, or trans-illumination, is for example known from WO 96/36273. In this specification it is proposed to trans-illuminate the related member with a light source. The observer is provided with a helmet having a telescope device using a filter and further night vision techniques. In US 6178340 a method for three dimensional infrared imaging of a part of member of a human being on base of reflectance is used. In other words camera and light source are used on the same side of the member to be observed and this results in the impossibility to observe arteries or veins which are somewhat deeper in the related member.

The present invention aims to provide a relatively simple instrument which is easy to use, cheap to produce and improves the procedure of vessel puncture.

This aim is realized with an apparatus having the features of claim 1.

In contrast to WO 96/36273 a display is used being spaced from the observer. In this way the related member and display are about the same distance from the eyes of the observer with regard to focusing of the eyes of the observer. This means that in one glance the observer can both observe the related member and the display.

It is possible to use cheaply obtained components for the apparatus according to the invention. The light source can comprise an IR led preferably provided with a strap for connecting to the hand or arm of the observer or patient in order to manipulate it in the correct position relative to the member to be observed. The camera can comprise any camera such as a usual CCD camera. The display can comprise any display such as an LCD display.

According to a preferred embodiment of the invention the display is fixedly connected to the camera in the position of use. More preferable display and camera can be freely manipulated together and to that end they are mounted on the extremity of a swivel arm. After placing the related member in the desired position the combination camera and display can be moved together such as to obtain an optimum image of the member and more particular the arteries and veins thereof. If the required position is reached the observer can take the related measure for example puncturing the artery or vein. More preferable the swivel arm together with camera, display and other auxiliaries is weight balanced i.e. is stable in any position in which it is placed. This means that the observer manipulates the combination of camera and display to the desired position with one hand whilst preferably the other hand is used to manipulate the light source. It is of course possible that the light source is present in a support on which the member such as a hand is laid or connected to the patient. After correctly positioning of the camera/display the observer no longer engages the swivel arm and can use his hand to operate a syringe or other means for treating the related extremity.

These measures make it possible that in one position of the observer without substantial movement of his head, he is able to both see the display and the place where for example a puncture should be effected. Also the distance between the display and the observer and between the observer and the place to examine (puncture) is such that no substantial refocussing by the observer is necessary when his view is changed from the display to the place to be examined and the member of the living being. In other words only a movement of the eyes of the observer is necessary to go to the place to be examined from the display and vice versa.

The apparatus according to the invention can be used for example with incubators wherein display and camera can be kept outside the incubator in which a child is present.

For operation of camera and display mounted to the swivel arm preferably a control grip is provided for engagement by the hand of the observer. If any operating means such as adjusting means for focusing of the camera or controlling the intensity of the light emerging from the infra red source, such control means can be provided on the grip and controlled by a single hand.

According to a preferred embodiment of the invention the swivel arm is embodied such that the mounting of the camera and display will always have the same orientation (preferably vertical) irrespective of the position of the swivel arm. This can for example be effected by constructing the swivel arm from a number of sub-arms producing a parallelogram structure. In this way parallax can be further avoided.

The invention also relates to a method for determining the position of a vein or artery in a member of a living being by an observer, comprising trans-illumination of said member with an IR light beam from one side of said member to the other side thereof, scanning said opposite side of said member with an IR sensitive camera and producing a visible image of said camera scanned area, wherein producing of a visible image comprises showing said image on a display spaced at least 30 cm (b) from the eyes of the observer.

More particular the position of the observer, display and vein or artery in said member, is such that the line of sight of the observer to the display substantially corresponds to the line of sight of the observer to said vein or artery.

Preferred embodiments of the invention will be further elucidated referring to the enclosed drawings, wherein:
Figure 1 shows the general layout of the apparatus according to a first embodiment of the invention;
Figure 2 shows a detail of positioning of camera and display relative to each other;
Figure 3 shows the use of an apparatus according to the invention in a first step;
Figure 4 shows the use of a device in a subsequent step and
Figure 5 shows schematically a further embodiment of the swivel arm of the invention.

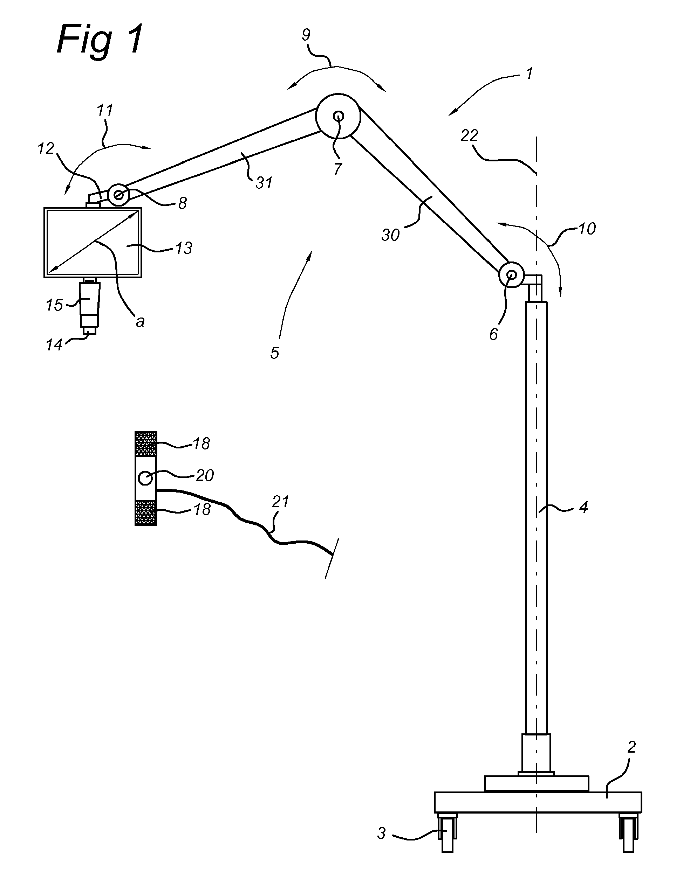

In Figure 1 the apparatus according to the invention is generally indicated by 1. This apparatus comprises a lower frame 2 which can include a weight for stabilizing the arm provided thereon. The lower frame is provided with wheels 3 which can optionally be blocked. Column 4 extends from the lower frame and arm 5 is swivelable received in upper part of column 4. The swivel axis is indicated by 22. Arm 5 comprises two arm portions 30 and 31. Arm portion 30 is provided with pivot 6 at one extremity thereof and a pivot 7 at the other extremity thereof. Arm 31 is provided with pivot 7 and pivot 8. Articulation of pivot 6 is in the direction of arrow 10. Articulation of pivot 7 is in the direction of arrow 9 whilst movement of pivot 8 is in the direction of arrow 11. Pivot 8 comprises a mounting 12 to which a display 13, grip 15 and camera 14 are mounted. Springs and/or other weight balancing means are provided such that in any pivotal position of the pivots 6, 7 and 8 there is not bias for movement of mounting 12 with camera and display.

Through the combination of these pivots 6, 7, 8 and swiveling around axis 22 any position of the camera/display combination can be made in x, y and z direction.

Of course it is possible not to use a column 4 but to use an arm 5 connected to a wall, table or other piece of furniture.

Apparatus 1 also comprises an IR source such as an IR led 20 being provided with straps 18 which can be connected to each other through e.g. a Velcro connection. This IR source has a finger mounting to receive the IR source on one of the fingers of the observer. The mounting can be connected to the finger in any possible way. The above straps are used as example but clipping or a spring connection to the related finger (which can be any finger) could be effected. 21 Shows a possible feed for the infrared source 20 but of course a battery operated source can be used. The support in which the infrared source is integrated is also possible. The IR light used can be any infrared light and is preferably near infra light.

For the camera and display easy obtainable components can be used such as a CCD camera and LCD display.

The infrared source is preferably a LED light source. Four LEDs are used in this example. Those LEDs do not generate substantial heat such the related member to be examined can be firmly pushed there against.

The diagonal of the LCD display has been indicated by a and is at least 6 cm.

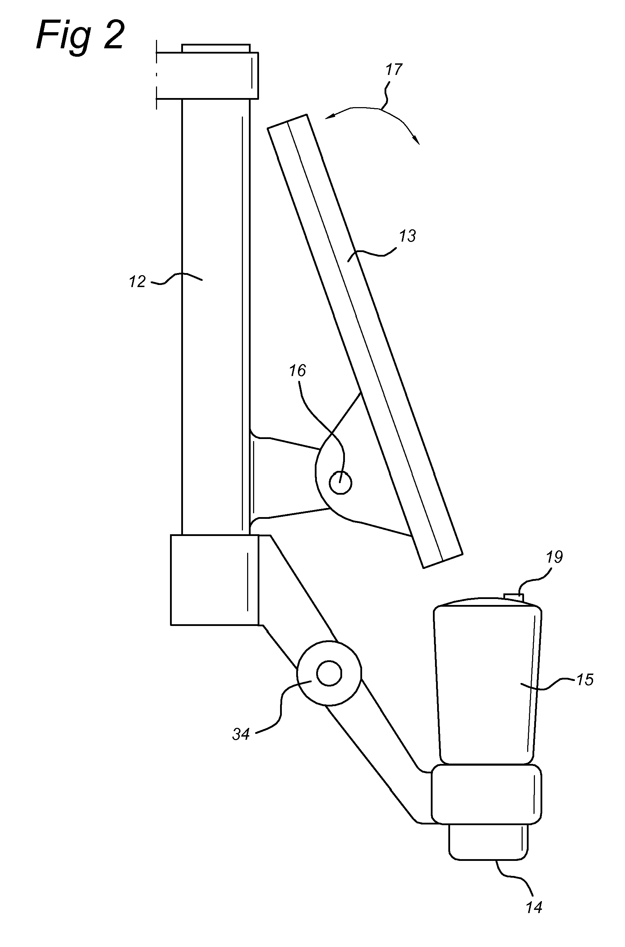

Figure 2 shows a detail of mounting 12. It is clear that display 13 is mounted through an articulated connection with mounting 12. This allows for adjustment of the display 13 relative to the mounting 12. However this adjustment is generally only made at taking the position of the observer and after his position has been taken (seated) no further adjustment is necessary. The corresponding pivot 34 is provided between grip 15/camera 14 and mounting 12. Also here adjustment is only made before starting the observation procedure. Because the device is directed through grip 15 the friction in pivot 34 to be overcome should be relatively high so that when grip 15 is engaged with a relatively low force mounting 12 will exactly follow. Only if grip 15 is firmly engaged with a tilting movement relative movement can be effected through pivot 34. Grip 15 is provided with operating means such as an adjustment mechanism for either focusing camera 14 or adjusting the intensity of the infrared source 20. Further control means may be provided to this end.

From figure 1 and 2 it is clear that through movement of grip 15 the combination of camera and display can be put in any position and orientation relative to the observer.

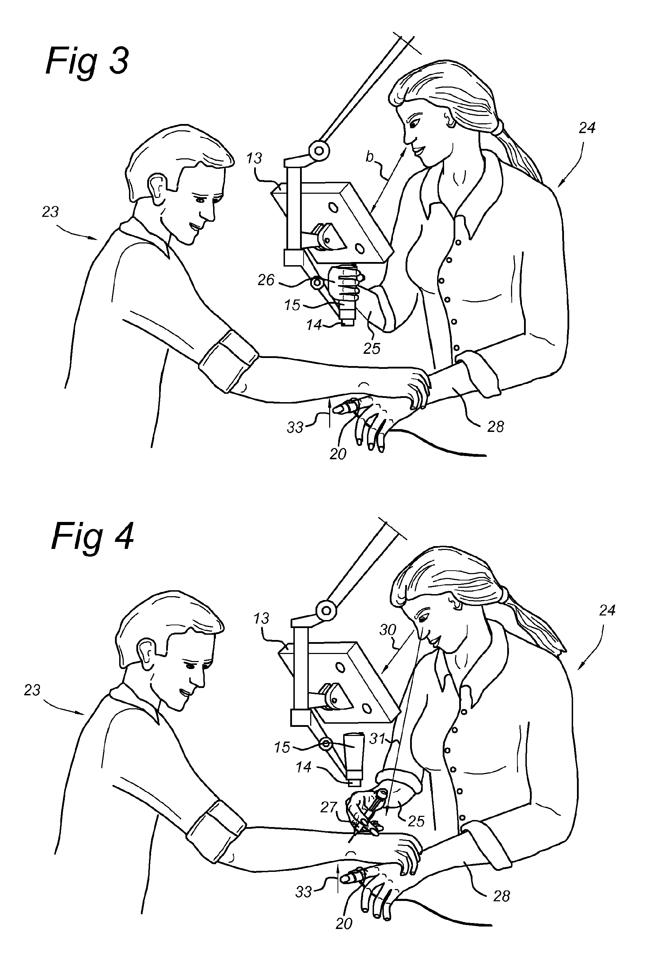

This is further elucidated in figure 3 and 4. Figure 3 shows an observer 24 and a patient 23. It is assumed that the hand or arm of the patient has to be punctured. To that end the infrared source 20 is mounted with straps 18 or other means to a finger of the left hand of the observer or in any other position on the hand or arm 28 of the observer 24 and manipulated against the underside of the arm or hand of the patient (arrow 33). The right arm 25 are more particular the right hand of the observer 24 is used to manipulate the combination of camera 14 and display 13 above the spot of the extremity of the patient 23 which should be observed. This is effected by engagement of grip 15. The distance of the observer 24 to the display is indicated by b and is more than 30 cm. In this way at the same glance the observer can both watch the display and the related spot itself on the arm or hand of the patient 23. This is indicated in figure 4 wherein the line of sight between the observer and the display is indicated by 30 and the line of sight between the eyes of the observer and the arm to be punctured is indicated by 31. It is clear that only a changed position of the eyes it sufficient to go from the display to the arm and vice versa.

Once the display/camera is in the correct position the observer no longer engages grip 15. Because of the weight balance nature of arm 5 the display/camera will remain in the last position and the observer can use his right arm/hand to execute the related treatment such as realizing a puncture in a vein or artery.

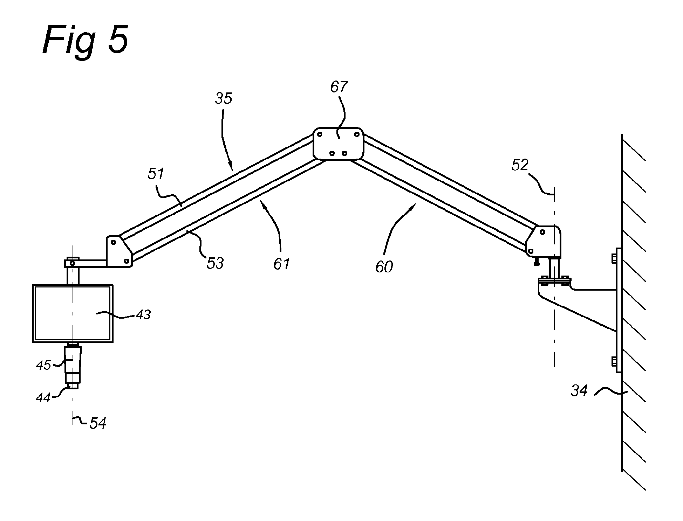

Figure 5 shows a further embodiment of the invention wherein a different swivel arm 35 is used. As in the previous example this a weight balanced arm and balancing can be effected through the use of springs (not shown), weight, and/or friction discs. Arm 35 is mounted to wall 34. Arm 35 comprises arm portions 60 and 61 being connected through a pivot plate 67. Each arm portion comprises two parallel arms 51 and 53. In this embodiment the structure is such that at displacement of the assembly of camera 44, grip 45 and display 43 axis 54 will always remain parallel to axis 52 around which rotation is possible. Usually this will mean that axis 54 is always substantially vertical and in this way parallax at observation of the related member is prevented as much as possible.

It should be realized that the description of the figures relates to a preferred embodiment but after reading the above a person skilled in the art will immediately images further variants which are within the scope of protection of the attended claims.

## Claims

1. Apparatus (1) for determining the position of a vein or artery in a member of a living being, comprising an IR source (20) adapted to be placed at one side of a member of a living being and transferring light through such a member, a camera (14) adapted to be placed at another side of a member of a living being, said other side being opposite to one side of a member of a living being and sensitive for radiation from said IR source, observation means (13) for observing the image of said camera, **characterized in that**, said observation means comprise a rectangular display having an effective diagonal of at least 6 cm.

2. Apparatus according to claim 1, wherein said display is fixedly connected to said camera in the position of use.

3. Apparatus according to one of the preceding claims, comprising a swivel arm (5, 35) wherein said camera and display are mounted to the extremity of said swivel arm.

4. Apparatus according to claim 3, wherein said swivel arm is weight balanced.

5. Apparatus according to one of the preceding claims, wherein said camera is mounted near said display.

6. Apparatus according to one of the preceding claims, wherein said light source comprises an IR led.

7. Apparatus according to one of the preceding claims, comprising a control grip (15), wherein the camera (14) and display (13) are mounted to the control grip.

8. Apparatus according to one of the preceding claims, wherein the control grip (15) comprises adjustment means (19) for controlling the intensity of the light source and/or the focus of the camera.

9. Apparatus according to one of the preceding claims, wherein the IR source comprises a strap (26).

10. Apparatus according to one of the preceding claims, wherein the IR source comprises a finger mounting.

11. Apparatus according to one of the preceding claims wherein the swivel arm (35) is embodied such that at swivelling the orientation of the camera and display is substantially not changed.

12. Method for determining the position of a vein or artery in a member of a living being by an observer, comprising trans-illumination of said member with an IR light beam from one side of said member to the opposite side thereof, scanning said opposite side of said member with an IR sensitive camera and producing a visible image of said camera scanned area, **characterized in that**, producing of a visible image comprising showing said image on a display (13) spaced at least 30 cm (b) from the eyes of the observer (24).

13. Method according to claim 12, wherein said camera and display being freely manoeuverable relative to said member.

14. Method according to one of the claims 12 or 13, wherein the position of the observer, display and vein or artery in said member, is such that the line of sight (30) of the observer (24) to the display (13) substantially corresponds to the line of sight (31) of the observer to said vein or artery.

15. Method for introducing or removing a substance in/from a vein or artery of a living being, comprising determining the position of said vein or artery according to one of the claims 12-14 engaging a syringe (27) by the observer (24), entering said syringe in said member while simultaneously said member is observed with said IR light.

16. Method according to claim 15, wherein said member comprises an extremity.

17. Method to one of the claims 12-16, wherein said IR light beam originates from a source which is firmly placed against said member.
